(19) Europäisches Patentamt
European Patent Office
Office européen des brevets

(11) **EP 2 673 248 B1**

(12) **EUROPEAN PATENT SPECIFICATION**

(45) Date of publication and mention
of the grant of the patent:
**28.12.2016 Bulletin 2016/52**

(51) Int Cl.:
***C07C 7/163*** *(2006.01)*   ***C07C 7/167*** *(2006.01)*
***C07C 5/09*** *(2006.01)*

(21) Application number: **12704583.9**

(22) Date of filing: **02.02.2012**

(86) International application number:
**PCT/US2012/023625**

(87) International publication number:
**WO 2012/109085 (16.08.2012 Gazette 2012/33)**

(54) **LIQUID PHASE HYDROGENATION OF ALKYNES**

HYDRIERUNG VON ALKYNEN IN DER FLÜSSIGPHASE

HYDROGÉNATION D'ALCYNES EN PHASE LIQUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.02.2011 US 201113024944**

(43) Date of publication of application:
**18.12.2013 Bulletin 2013/51**

(73) Proprietor: **Saudi Basic Industries Corporation
Riyadh 11422 (SA)**

(72) Inventors:
• **MAMEDOV, Edouard
Houston, Texas 77094 (US)**
• **HORN, Jillian
La Plata, Maryland 20646 (US)**
• **ALLMAN, Jim
Sugar Land, Texas 77479 (US)**
• **ARAUJO, Armando
Houston, Texas 77018 (US)**
• **SIMMONS, Meghann
Katy, Texas 77494 (US)**

(74) Representative: **Modiano, Micaela Nadia
Modiano & Partners (DE)
Thierschstrasse 11
80538 München (DE)**

(56) References cited:
**EP-A1- 0 689 872   WO-A1-00/64846**

• **HONG ET AL: "Preparation of novel titania
supported palladium catalysts for selective
hydrogenation of acetylene to ethylene",
CATALYSIS COMMUNICATIONS, ELSEVIER
SCIENCE, AMSTERDAM, NL, vol. 8, no. 3, 1 March
2007 (2007-03-01), pages 593-597, XP022134699,
ISSN: 1566-7367, DOI:
10.1016/J.CATCOM.2006.08.010**
• **KIM W J ET AL: "Effect of potassium addition on
the properties of a TiO2-modified Pd catalyst for
the selective hydrogenation of acetylene",
APPLIED CATALYSIS A: GENERAL, ELSEVIER
SCIENCE, AMSTERDAM, NL, vol. 268, no. 1-2, 10
August 2004 (2004-08-10), pages 77-82,
XP004512936, ISSN: 0926-860X, DOI:
10.1016/J.APCATA.2004.03.025**

**Description**

BACKGROUND OF THE INVENTION

FIELD OF THE INVENTION:

This invention relates to a process for liquid-phase selective hydrogenation of unsaturated hydrocarbons, such as acetylenes, to less unsaturated analogs, such as olefins, with a rnulticomponent catalyst comprising Pd on a titania support. The catalyst may also comprise one or more of a particular metal modifier. The process may also comprise the absence or presence of a carbon monoxide co-feed.

DESCRIPTION OF THE PRIOR ART:

Hydrogenation of alkynes and/or multifunctional alkenes to compounds containing only one alkene group is an important industrial process and is discussed widely in the patent literature. Usually it is performed in the gas phase in the presence of supported palladium catalysts. There is a large amount of literature covering both practical and fundamental aspects of the gas-phase hydrogenation of acetylene to ethylene.

In contrast, there are fewer examples of acetylene hydrogenation in the liquid phase.

U.S. Patent 4,128,595 describes a process in which an acetylene-rich gas is contacted with hydrogen via an inert saturated liquid hydrocarbon stream with hydrogenation occurring over palladium-on-alumina catalyst wherein palladium loading ranges from 0.01 to 10 weight percent. As inert saturated liquid hydrocarbons, various hexanes, decanes and decalin can be used. The process requires the acetylene-containing gas and inert liquid to be fed concurrently into the top of a trickle bed reactor because acetylene solubility in the saturated hydrocarbon liquid is poor at reaction conditions. This patent teaches that polar solvents, such as dimethylformamide (DMF), provide lower selectivity to ethylene and can cause rapid deactivation of the catalyst.

U.S. Patent 4,571,442 discloses a process for selective hydrogenation of acetylene in a mixture with ethylene, which is passed through a palladium-on-alumina catalyst in the presence of a liquid phase. This liquid phase contains 15 to 100% by weight of at least one aromatic hydrocarbon and at least one amine compound dissolved in the liquid phase and selected from the group consisting of primary amines, secondary amines and polyamines, so as to increase acetylene conversion to ethylene. The concentration of amine compound is claimed to be from 0.1 to 1% by weight of hydrocarbon in the liquid phase. In examples reported in the patent, acetylene was present at levels of 1wt% in a stream of ethylene.

U.S. Patent 5,059,732 describes a process to hydrogenate effluent from a steam cracker containing 0.4wt% acetylene, with hydrogen in the presence of a palladium or other noble metal catalyst by use of a gasoline cut as an inert liquid phase. The liquid phase containing at least 25% by weight of aromatic hydrocarbons is introduced into the hydrogenation reactor concurrently with the feed to be hydrogenated. The aromatics are believed to slow down the formation of green oil thus increasing catalyst life.

U.S. Patent 6,867,338 claims a process for the liquid-phase hydrogenation of acetylenes and dienes in a gaseous stream exiting the quench system of a steam cracker. To eliminate the possibility of a runaway reaction, the said stream is fed concurrently with the unspecified liquid to the top of a down flow boiling point reactor containing a palladium-on-alumina catalyst. The amount of the liquid being recycled is maintained sufficient to ensure that the catalyst is fully wetted at all positions within the reactor. The reactor is operated at the boiling point of the mixture in the reactor and the heat of reaction is absorbed by the boiling liquid.

Russian Patent RU 2,169,755 discloses a two-step process for converting natural gas to ethylene. At first, natural gas is pyrolized under the conditions of hydrogen plasma to give the product containing up to 15wt% acetylene. Acetylene is then catalytically hydrogenated to ethylene in the organic liquid the boiling point of which is close to the temperature of acetylene hydrogenation. In the example described in the patent, acetylene was hydrogenated on two-layer catalyst that consisted of kieselguhr-supported nickel and alumina-supported palladium, and kerosene-gasoline cut was used as a liquid phase.

The above-cited patents suggest that the gaseous acetylene undergoes hydrogenation in the presence of non-polar hydrocarbon liquid which mainly absorbs reaction heat and washes out some green oil from the catalyst surface.

U.S. Patent 6,358,399 proposes to first separate the $C_2^+$ hydrocarbons from a hydrocarbon steam-cracking effluent by absorbing them in an absorption column with a cooled solvent and then hydrogenate the liquid phase in the presence of hydrogen and a hydrogenation catalyst which can be alumina-supported palladium optionally containing at least one metal of Group IB. As a solvent, toluene, pentane, hexane, a toluene-benzene mixture and a cyclohexane-toluene mixture can be used. The process is claimed to be effective in eliminating all of the triple-bond compounds and the diene compounds present in a hydrocarbon steam-cracking effluent.

U.S. Patent 7,045,670 also proposes to hydrogenate the acetylene dissolved in a polar liquid. Based on this idea, the inventors claim a selective hydrogenation process which comprises (i) acetylene absorption in a liquid absorbent, (ii) contacting the dissolved acetylene with a hydrogenation catalyst in the presence of hydrogen-containing gas stream at hydrogenation conditions, and (iii) separation of the absorbent from the product stream to recover a product. Herein the extraction of acetylene from the gaseous stream can be performed in a specially designed unit or in an extraction-hydrogenation zone located within reactor. In latter embodiment, extraction and hydrogenation steps are performed in situ. Non-hydrocarbon polar solvents, such as n-methyl-2-pyrrolidone (NMP), acetone, tetrahydrofuran (THF), dimethylsulfoxide (DMSO) and monomethylamine (MMA), are used to absorb the acetylene. For instance, NMP is able to dissolve up to 5 wt% acetylene. The liquid stream may also contain a high boiling additive that is combined with the polar solvent before feeding it to reactor. It exhibits very low vapor pressure allowing the acetylene to remain with the solvent during the hydrogenation process. Examples of suitable additives include different phosphines, thiophines, pyridines and anilines. The catalyst compositions for this process are claimed to comprise a supported Group VIII metal, such as palladium, and a second metal selected from the group consisting of Group IB metals, such as Ag, Group IIB metals, such as Zn, Group IIIA metals, such as In and Ga, Group VIIB metals, such as Mn, and combinations thereof. The support is claimed to be a particulate alumina which is impregnated by incipient wetness impregnation with an aqueous solution of soluble salts of metals selected from the listed groups. A promoted catalyst is made by a method which comprises applying a first metal, usually palladium, to a support, applying a second metal to the support already coated with a first metal, followed by drying and calcining first and second metal-coated support to obtain a precursor and then reducing the precursor with a $H_2$-CO mixture to provide 0.1-1.0 wt% of the first metal and 0.05-1.2 wt% of the second metal to final weight of the catalyst. It is claimed also that the first and second metals can be applied to the support concurrently.

All above-listed patents describe liquid-phase hydrogenation of acetylene on conventional powdered catalysts which are metals supported on an inorganic material. The majority of catalysts are comprised of palladium supported on alumina. There are several examples in the literature where liquid-phase hydrogenation has been carried out using structured catalysts. R.K. Edvinsoon et al. published a paper in Ind. Eng. Chem. Res. 1995, 34, 94-100, where they reported the results of studying acetylene liquid-phase hydrogenation on monolithic catalysts prepared by depositing palladium on $\alpha$-alumina monolith as well as on cordierite monoliths washcoated with $\alpha$-alumina. Activity and selectivity of these catalysts dropped during the first 60 hours of testing and then leveled out. It was found that n-heptane was capable of removing green oil from the catalyst.

Better performance of the Pd-monolith down flow bubble column reactor in comparison with slurry and fixed bed operation mode was reported also for the liquid-phase hydrogenation of butyne-1,4-diol to cis-2-butene-1,4-diol (see H. Marwan and J.M. Winterbottom, Catalysis Today 2004 97, 325-330). The advantages of monolithic reactor were smaller reaction volume and better transport properties that intensified reaction rate.

As is apparent, mostly alumina-supported Pd catalysts have been claimed thus far to be efficient in the liquid-phase hydrogenation of alkynes to alkenes. This invention shows that Pd supported on other oxidic material, such as titania, can also catalyze liquid-phase hydrogenation of acetylene to ethylene with high conversion and selectivity. Moreover, many of these catalysts are more active and selective than their alumina-supported analogues.

U.S. Patent no. 7,220,701 discloses an alkyne/alkadiene selective hydrogenation process with operating parameters including a temperature of from about 20°C to about 150°C, such as from about 30°C to about 100°C, a pressure of from 690 kPa to 4100 kPa (about 100 psig to about 580 psig), such as from 1400 kPa to 3400 kPa about 200 psig to about 440 psig), a $H_2/C_2H_2$ molar feed ratio of from about 1 to about 1000, such as of from about 1.1 to about 800 and, in the vapor phase, a GHSV from about 100 to about 20,000, such as from about 500 to about 15,000 or, in the liquid phase, an LHSV of 0.1 to 100, such as from 1 to 25.

The catalyst contains rhodium and indium on a support of carbon, silicon nitride, silicon carbide, boron nitride, magnesium silicate, bentonite, zeolites, metal alloys, zirconia, alumina, silica, silica-alumina, ceria-alumina, aluminates (such as aluminates of Groups 1 and 2 of the Periodic Table of Elements), and magnesium oxide-silicon oxide mixtures, preferably alumina, zirconia and ceria-alumina. Carbon monoxide may be present as an impurity up to 1 ppm.

U.S. Patent no. 7,301,062 discloses a process for selective hydrogenation of alkynes and/or dienes in either gas, liquid or mixed phase with a catalyst of palladium and at least Group IB metal, such as silver, copper or gold, impregnated on an inorganic support having a surface area from about 2 to about 20 $m^2$/g and a pore volume greater than about 0.4 cc/g, wherein at least about 90% of the pore volume is contained in pores with pore diameters larger than about 500 angstroms and wherein the pore volume of the pores with a pore diameter from about 500 to about 1,000 angstroms comprise from about 1 to about 2% of the total pore volume. The support is a low surface area catalyst carrier, such as alumina, silica-alumina, zinc oxide, nickel spinel, titania, zirconia, ceria, chromia-alumina, magnesium oxide, cerium oxide and mixtures thereof, preferably alumina. Trace quantities of carbon monoxide may be present in the feed. Increased concentration of carbon monoxide results in lower conversion.

U.S. Patent No. 6,388,150 discloses a process of selective hydrogenation of an impurity, such as acetylene compound, dienes and mixtures thereof, in a hydrocarbon feed of at least one monoolefin. The catalyst is a noble metal with an optional metal promoter supported on a mesh-like non-catalytic material of fibers or wires in one or more layers.

Carbon monoxide may be added to the feed as a selectivity promoter.

U.S. Patent No. 4,691,070 discloses a catalyst of palladium for the hydrogenation of a diolefin with a cocatalyst of ruthenium, rhodium, cobalt or rhenium, said catalyst and cocatalyst on a support of a non-acidic inorganic compound having a surface area of not more than 100 $m^2$/g. For a liquid phase reaction, the diolefin should be used in the form of a dilute solution to prevent dimerization. A continuous gaseous phase reaction is preferred.

WO 00/64846 discloses a hydrogenation process in which a hydrocarbon-containing fluid containing a highly unsaturated hydrocarbon such as a diolefin and/or an alkyne is contacted, in the presence of hydrogen and a sulfur impurity such as a sulfur compound, with a catalyst composition containing-palladium and titanium dioxide in a hydrogenation zone under a condition effective to convert the highly unsaturated hydrocarbon to a less unsaturated hydrocarbon.

## SUMMARY OF THE INVENTION

The present invention is for a process for selective hydrogenation of acetylene, with a catalyst comprised of Pd and, optionally, at least one metal from the IA, IB, IIIA, IIIB, VB or VIIIB Groups, where the metals are deposited on a catalyst support. The Group IA metal may be lithium, potassium, or cesium, preferably potassium. The Group IB metal may be copper, silver, or gold, preferably gold. The Group IIIA may be boron. The Group IIIB metal may be lanthanides, preferably lanthanum or cerium. The Group VB metal may be vanadium, niobium, or tantalum, preferably vanadium. The Group VIIIB metal may be cobalt, nickel, ruthenium, rhodium, or platinum, preferably nickel or ruthenium. The catalyst support is an inorganic material, such as titania. The process is liquid phase selective hydrogenation of acetylene to ethylene by contacting an acetylene-containing liquid stream of solvent/absorbent with a hydrogen-containing gaseous stream in the presence of the catalyst of the invention. There may be an absence or presence of carbon monoxide in the acetylene-containing feedstream.

## BRIEF DESCRIPTION OF THE_DRAWINGS

A more complete appreciation of the invention and many of the attendant advantages thereof will be readily understood by reference to the following detailed description when considered in connection with the accompanying drawing:

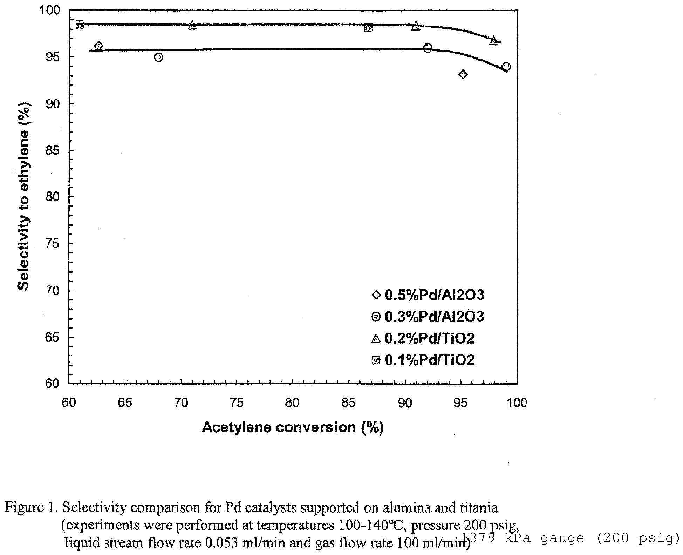

Figure 1 compares selectivities for Pd catalysts supported on alumina and on titania at temperatures of 100-140°C, pressure of 1379 kPa gauge (200 psig), liquid stream flow rate of 0.053 ml/min and gas flow rate of 100 ml/min.

## DETAILED DESCRIPTION OF THE INVENTION

The present invention comprises an improved hydrogenation process. The hydrogenation process pertains to the hydrogenation of acetylene to ethylene wherein the feed comprises dimethylformamide. The hydrogenation is carried out in the liquid phase which is also used as a solvent/absorbent for the acetylene. The hydrogenation may be carried out in any of a fixed bed reactor, trickle bed reactor, slurry bubble column reactor, catalytic distillation reactor or any combinations of these.

This invention is useful in the selective hydrogenation of acetylene to ethylene wherein the acetylene is absorbed from a gas stream by use of a non-hydrocarbon absorbent liquid to provide a reactant stream. Thereafter the reactant stream containing acetylene contacts a hydrogen-containing gaseous stream in the presence of hydrogenation catalyst. Carbon monoxide may be contained in a stream comprising hydrogen or may be fed concurrently or simultaneously with the hydrogen required for hydrogenation. The hydrogenation catalyst disclosed in this invention is monometallic or bimetallic catalyst supported on porous material such as titania, zirconia, silica, alumina, silica-alumina, etc. The preferred support is titania which has total pore volume of at least 0.25 $cm^3$/g and the average pore diameter of larger than 150 Å. The BET surface area is in a range of from 30 to 100 $m^2$/g The physical shapes may be spheres, pellets, granules, or extrudates whose size is in a range of from 1 to 6 mm.

The preferred support disclosed in this invention can be prepared by a number of techniques well known to those skilled in the art of preparing porous oxidic materials. Various forms of this support are also available in the market place. To prepare the support with a given texture (surface area, pore volume, etc.), shaped raw or ready material is calcined at a temperature in a range of from about 400 to 1000°C. The final calcination temperature is determined by the physical properties of support to obtain the best performance of catalyst for the specific hydrogenation process. Table 1 lists physical properties of the supports used in this invention for preparation of hydrogenation catalysts.

| Table 1. Titania supports for hydrogenation catalysts | | | | |
|---|---|---|---|---|
| Support supplier | Product number | Surface area ($m^2/g$) | Pore volume ($cm^3/g$) | Pore diameter (Å) |
| St Gobain/Norpro | ST31119 | 39 | 0.26 | 270 |
| | XT25384 | 43 | 0.35 | 332 |
| ASM Catalysts | Mirkat 435T | 98 | 0.48 | 194 |

Desired elements can be deposited on a support by various techniques such as solution impregnation using rotary evaporation, incipient pore impregnation, spray-coating impregnation using atomizer, vapor deposition, co-precipitation, etc. The preferred technique in this invention is incipient pore impregnation. Most of the experimental catalysts described below involve dissolving the salts of metals in the amount of water required to just fill the internal pores of the catalyst support. In this method, the volume of the liquid impregnation solution for a given amount of support is important. The desired volume of the solution should not exceed tile total volume of the support, preferably being from 90 to 95% of the pore volume of the support.

Depending on the objective of the specific hydrogenation reaction, which determines what elements and how many are needed in the catalyst, the preparation of supported catalyst may be carried out using a single or multi step procedure. When Pd is one of the desired two components of the catalyst, the preparation may be carried out in one or two steps. For the single step preparation, a mixed solution of Pd and second element compounds in water or organic solvent is prepared and impregnated on a support to incipient wetness. The impregnation product is dried with a hot gas such as air or nitrogen at a temperature in a range of 60 to 250°C, preferably in a range of 100 to 200°C. The dried impregnation product is normally calcined in air at a temperature of 250 to 600°C, preferably at a temperature of 300 to 500°C. The calcined material is then reduced in a flow of hydrogen (5 volume % H2 gas in N2) at a temperature in a range of 250 to 600°C, preferably in a range of 300 to 500°C: to give a supported bimetallic catalyst ready to use. If two-step preparation is desired, a support is first impregnated to incipient wetness with a solution of a Pd compound followed by drying, calcination and reduction at appropriate temperatures described earlier. Thus prepared supported Pd catalyst is then impregnated with a solution of second element compound, dried, calcined and reduced under the conditions similar to the conditions of the first step of preparation. Another alternative two-step preparation suggests a support first to be loaded with a second element and then with palladium. In this preparation, the drying, calcining and reducing procedures are also conducted twice.

The aqueous solution of water-soluble palladium compound used in the process of this invention may include aqueous solution of any suitable palladium compound such as palladium (II) nitrate, palladium (II) chloride, palladium (II) sulfate, palladium (II) ammoniachloride, potassium tetrachloropalladium (II), etc. More preferable are palladium (II) chloride and palladium (II) nitrate, the most preferable is palladium (II) chloride. The aqueous solutions of second metal compounds may include aqueous solutions of ally water-soluble compounds such as nitrates, chlorides, hydroxides and others. The most frequent used compounds of second metals are nitrates and chlorides.

The quantity of palladium compound to be employed is such as to provide in the ha1 catalyst a Pd loading of from 0.05 to 1% by weight, more preferably from 0.1 to 0.5% by weight. The quantity of second metal compound to be employed is such as to provide in the final catalyst a second metal loading that places it in the catalyst in a weight ratio to Pd in a range of from 0.5 to 2. 'The content of second metal in the catalyst normally ranges from 0 to 0.5% by weight.

The present invention also includes the application of catalysts as described herein to selective conversion of acetylene to ethylene comprising the charging of a feedstream containing the acetylene to a single-pass continuous reactor containing the catalyst and operated at conditions conductive to hydrogenation. The acetylene may be a gas and the reactor may be operated such that the fluid media in the reactor is supercritical fluid phase form. The acetylene compound may alternatively be a liquid and distributed as a component of a stream wholly or mostly the gas state at reactor operating conditions such that the fluid media in the reactor is in a supercritical, or mixed phase form. Alternatively, acetylene may be a liquid and distributed as a component of a stream wholly or mostly in the liquid state at reactor operating conditions such that the fluid media in the reactor is in the liquid, supercritical, or mixed phase form. Also, the may be a gas at reactor operating conditions and distributed as a component of a stream wholly or mostly in the liquid state such that the fluid media in the reactor is in a liquid, supercritical, or mixed phase form, The present invention further includes a method for screening or evaluating the suitability of catalysts for selective hydrogenation of acetylene to ethylene, particularly for screening the catalysts on the basis of estimated conversion and selectivity. This method provides steps including (among others) preparing a reactant stream comprising acetylene in a liquid absorbent/solvent, contacting a liquid reactant stream and a gaseous hydrogen/carbon monoxide stream with the test catalyst, and measuring product concentrations in the steady-state liquid phase hydrogenation of acetylene, from which catalyst performance can be evaluated. To more clearly illustrate the present invention, a number of examples are presented below.

Comparative Example 1

The commercial eggshell type 0.3 wt% $Pd/Al_2Q_3$ catalyst from Engelhard was used in this invention as a reference catalyst. The average size of catalyst extrudates (product number SE01482) was 3 mm diameter and 3.5 mm long. For evaluating catalyst behavior in the microreactor, the extrudates were crushed and double-screened between 16 and 18 mesh

(U.S. sieve series) screens, thus providing particles with a minimum dimension of about 1 mm. Sieved catalyst was neither reduced nor activated before evaluating its catalytic behavior.

Comparative Example 2

The commercial eggshell type 0.5 wt% $Pd/Al_2O_3$ catalyst originally available from Strem Chemicals was used in this invention as another reference catalyst. The catalyst was received as "reduced" in the form of 3 mm cylinders (product number B3510044). For testing in the microreactor, catalyst was crushed and double-screened between 16 and 18 mesh (USS) screens to get particles with a minimum dimension of about 1 mm. Sieved catalyst was not reduced or activated before evaluating its catalytic behavior.

Examples 1 and 2

Nominal compositions 0.15% $Pd/TiO_2$ (#1) and 0.2% $Pd/TiO_2$ (#2) by weight.
Catalysts were prepared using a titania support from Saint-Gobain/Norpro Company (product number ST31119). Support was received in the form of 3 mm pellets which were crushed and double-screened between 16 and 18 mesh screens to get roughly spherical particles with a diameter of about 1 mm. Two portions of sieved titania of 5 gram each were pore impregnated to incipient wetness with aqueous solutions of $PdCl_2$ of the corresponding concentration, dried in air flowing with the rate of 250 mL/min for 8 hours at 120°C and calcined at 300°C for 3 hours. The calcined material was then reduced at 300°C for 2 hours with a mixture of 5vol% hydrogen in nitrogen flowing at a rate of 250 mL/min to obtain the catalyst ready for testing.

Examples 3-6

Nominal compositions 0.1% $Pd/TiO_2$ (#3), 0.29'0 $Pd/TiO_2$ (#4), 0.3% $Pd/TiO_2$ (#5), and 0.5% $Pd/TiO_2$ (#6) by weight.
Catalysts were prepared using the same support as in Examples 1 and 2. Four portions of sieved support of 5 gram each were pore impregnated with aqueous solutions of $Pd(NO_3)_2$ of the corresponding concentration, dried in air flowing with the rate of 250 mL/min at 120°C for 8 hours and calcined at 300°C for 3 hours. Calcined materials were then reduced at 300°C for 2 hours with a mixture of 5vol% hydrogen in nitrogen flowing at a rate of 250 mL/min.

Example 7

Nominal composition 0.2% $Pd/TiO_2$ by weight.
Catalyst was prepared using a titania support XT25384 from Saint-Gobain/Norpro Company. Support 3 mm pellets were crushed and sieved to 16-18 mesh. 5 gram of sieved support was pore impregnated with aqueous solution of $Pd(NO_3)_2$ of the corresponding concentration, dried in air flowing with the rate of 250 mL/min at 120°C for 8 hours and calcined at 300°C for 3 hours. Calcined material was then reduced at 300°C for 2 hours with a mixture of 5vol% hydrogen in nitrogen flowing at a rate of 250 mL/min.

Example 8 and 9

Nominal compositions 0.2% $Pd/TiO_2$ (#8) and 0.5% $Pd/ZrO_2$ (#9) by weight.
Catalysts were prepared using a titania support from ASM Catalysts, LLC (product name Mirkat 435T). Pellets with average size of about 6 mm were crushed, sieved to 16-18 mesh and calcined in air at 700° for 3 hours. Two portions of calcined support of 5 gram each were impregnated to incipient wetness with aqueous solutions of $Pd(NO_3)_2$ of the corresponding concentration, dried in air flowing with the rate of 250 mL/min at 120°C for 8 hours and calcined at 300°C for 3 hours. After calcination the impregnated material was reduced at 300°C for 2 hours with a mixture of 5vol% hydrogen in nitrogen flowing at a rate of 250 mL/min.

Examples 10 and 11

Nominal compositions 0.1 % Pd -0.1 % K/TiO$_2$ (#10) and 0.1 % Pd -0.1 % Au/TiO$_2$ (#11) by weight.
Catalysts were prepared by a two-step method using a titania support ST31119 supplied by Saint-Gobain/Norpro Company. First, base 0.1 %Pd/TiO$_2$ catalyst was prepared using the procedure described above in Example 1. Two 5 gram portions of this catalyst were loaded with the corresponding second metal by utilizing pore impregnation technique. Given amounts of KNO$_3$ and HAuCl$_4$, both from Sigma Aldrich, were dissolved in de-ionized water and impregnated to incipient wetness onto the 0.1%Pd/TiO$_2$. The impregnation products were dried at 120°C for 8 hours and calcined at 300°C for 3 hours in air flowing with the rate of 250 mL/min. Calcined materials were then reduced at 300°C for 2 hours with a mixture of 5vol% hydrogen in nitrogen flowing at a rate of 250 mL/min.

Example 12

Nominal composition 0.2% Pd -0.2% Au/TiO$_2$ by weight.
Catalyst was prepared by impregnating aqueous solution of HAuCl$_4$ onto the base 0.2%Pd/TiO$_2$ catalyst prepared by the method described in Example 2. The impregnated material was dried at 120°C for 8 hours and calcined at 300°C for 3 hours in air flowing with the rate of 250 mL/min, and then reduced at 300°C for 2 hours with a mixture of 5vol% hydrogen in nitrogen flowing at a rate of 250 mL/min.

Examples 13-14

Nominal compositions 0.1 % Pd -0.1 % K/TiO$_2$ (#13) and 0.1 % Pd -0.1 % Au/TiO$_2$ (#14) by weight.
Catalysts were prepared using as a base the 0.1%Pd/TiO$_2$ prepared in Example 3. Two portions of base catalyst of 5 gram each were pore impregnated with aqueous solutions of KNO$_3$ and HAuCl4 of the corresponding concentrations: dried in air flowing with the rate of 250 mL/min at 120°C for 8 hours and calcined at 300°C for 3 hours. Calcined materials were reduced at 300°C for 2 hours with a mixture of 5vol% hydrogen in nitrogen flowing at a rate of 250 mL/min.

Examples 15 and 16

Nominal compositions 0.2% Pd -0.2% K/TiO$_2$ (#15) and 0.2% Pd -0.2% Au/TiO$_2$ (#16) by weight.
Catalysts were prepared by incipient wetness impregnation of the base 0.2%PdTiO$_2$ catalyst prepared in Example 4. Two 5 gram portions were pore impregnated with aqueous solutions of KNO$_3$ and HAuCl$_4$ of the corresponding concentrations, dried in air flowing with the rate of 250 mL/min at 120°C for 8 hours and calcined at 300°C for 3 hours. Calcined materials were reduced at 300°C for 2 hours with a mixture of 5vol% hydrogen in nitrogen flowing at a rate of 250 mL/min.

Examples 17-19

Nominal compositions 0.2% Pd -0.2% Ru/TiO$_2$, (#17): 0.2% Pd -0.2% La/TiO$_2$ (#18), and 0.2% Pd -0.2% V/TiO$_2$ (#19) by weight.
Catalysts were prepared by a two-step method using a titania support Mirkat 435T from ASM Catalysts, LLC. The support was crushed to 16-18 mesh and calcined at 700°C. First, base 0.2% Pd/TiO$_2$ catalyst was prepared using tile procedure described above in Example 8. This catalyst was split into three portions each of which was loaded with the corresponding second metal by employing pore impregnation technique. Samples of RuCl$_3$ from Strem Chemicals, La(NO$_3$)$_3$ from Sigma Aldrich, or VCl$_3$ from Sigma Aldrich were dissolved in de-ionized water and impregnated to incipient wetness onto the 0.2% Pd/TiO$_2$. The impregnation products were dried at 120°C for 8 hours and calcined at 300°C: for 3 hours in air flowing with the rate of 250 mL/min. Calcined materials were reduced at 300°C for 2 hours with a mixture of 5vol% hydrogen in nitrogen flowing at a rate of 250 mL/min.

Example 20

Nominal Composition 0.2% Pd -0.2% V/TiO$_2$ by weight.
Catalyst was prepared by the same two-step method used for the preparation of catalyst in Example 19. The only difference was that the support for this particular catalyst was titania X25384 supplied by Saint-Gobain/Norpro Company. The support was crushed to 16-18 mesh particles and loaded with palladium and vanadium in the way described in Example 19.

Example 21

Nominal composition 0.2% Pd -0.2% Au/TiO$_2$ by weight.

Catalyst was prepared in two steps using a titania support Mirkat 435T from ASM Catalysts, LLC. The support was crushed to 16-18 mesh and calcined at 700°C. At first, 0.2% Pd/TiO$_2$ catalyst was prepared following the procedure described in Example 8. Then 5 gram of this catalyst was impregnated to incipient wetness with the aqueous solution of HAuCl$_4$ (Sigma Aldrich) to add gold as a second metal. The impregnation product was dried at 120°Cfor 8 hours and calcined at 300°C for 3 hours in air flowing with tile rate of 250 mL/min. The reduction at 300°C for 2 hours with a mixture of 5vol% hydrogen in nitrogen flowing at a rate of 250 mL/min completed the preparation of this catalyst.

Example 22

Nominal composition 0.2% Pd -0.3% Au/TiO$_2$ by weight.

Catalyst was prepared in a single step by incipient wetness impregnation of a titania support XT25384 from Saint-Gobain/Norpro Company. 0.030 gram of HAuCl$_4$ was dissolved in 3 ml of de-ionized water and then 0.217 gram of Pd(NO$_3$)$_2$ was added to this solution. The resulting mixed solution was impregnated at room temperature onto 5 grams of titania particles of the 16-18 mesh size. The impregnation product was dried under air flowing at 250 mL/min for eight hours at 120°C and three hours at 300°C followed by the reduction with 5vol% hydrogen in nitrogen at 300°C for two hours.

Examples 23 and 24

Nominal compositions 0.2% Pd -0.3% Ce/TiO$_2$ (#23) and 0.2% Pd -0.2% Ni/TiO$_2$ (#24) by weight.

Catalysts were prepared by two-step method using a titania support XT25384 from Saint-Gobain/Norpro Company. In a first step, base 0.2%Pd/TiO$_2$ catalyst was prepared by the method described in Example 7. Then two 5 gram portions of this catalyst were separately impregnated to incipient wetness with aqueous solutions of Ce(N0$_3$)$_3$ ad Ni(N0$_3$)$_2$, both from Aldrich, to add cerium and nickel as second metals. The impregnated materials were dried under air flowing at 250 mL/min for eight hours at 120°C and three hours at 300°C, and then reduced with 5vol% hydrogen in nitrogen at 330°C for two hours.

All above-listed catalysts were tested in the hydrogenation of acetylene dissolved/absorbed in the n-methyl-2-pyrrolidone (NMP). An acetylene solution was first prepared by slowly bubbling pure acetylene at atmospheric pressure into a vessel containing NMP. Normally, 3-3.5 wt% acetylene was dissolved and absorbed in the NMP to provide a reactant liquid stream. The XMP saturated with acetylene was then drawn to vertically mounted down-flow 0.635 centimeter (¼") stainless steel reactor containing catalyst. Co-currently, a gas consisting of 5% CH$_4$, 5% CO and 90% H$_2$ volume was flowed under pressure and mixed with the liquid stream in the reactor to contact the catalyst. Depending on activity, the amount of catalyst loaded in the reactor was varied from 0.2 to 0.4 gram. The catalyst was placed in the reactor center section in a fixed bed configuration. All catalysts were tested at reactor pressure 1379 kPa gauge (200 psig) and temperatures 100 and 140°C. The liquid reactant flow rate was set at 0.053 ml/min the gaseous reactant stream was flowed at a rate of 100 ml/min. Upon exiting reactor, the reaction effluent cooled and entered the knock-out pot where a gas-liquid separation was made. The overhead gas was analyzed by a gas chromatograph (GC) using CH4 as an internal standard. It usually consisted of C$_2$ and C$_4$ hydrocarbons and CO which did not react. The liquid comprising NMP, C$_2$, C$_4$, and trace C$_6$ hydrocarbons was analyzed by another GC to obtain quantitative composition analysis. The mass balance closure for the results reported in this invention ranged from 98 to 103%.

Using the results of GC analyses, acetylene conversions and ethylene selectivities were calculated for purposes of comparing the performance of the catalyst formulations tested. The actual % conversions of acetylene were estimated according to the equation:

$$X(\% / mg) = \frac{(C_2H_4) + (C_2H_6) + 2(C_4H_6) + 2(C_4H_8) + 2(C_4H_{10})}{(C_2H_2) + (C_2H_4) + (C_2H_6) + 2(C_4H_6) + 2(C_4H_8) + 2(C_4H_{10})} \times 100$$

where (C$_2$H$_2$) is acetylene mole % in the reaction product, i.e. unconverted acetylene. Also acetylene conversions per unit weight of palladium were estimated by normalizing acetylene actual conversion to the weight of palladium in a catalyst charge:

$$X(\% / mg) = \frac{Actual Conversion(\%)}{Palladium Weight(mg)}$$

This normalized conversion of acetylene defined specific activity of supported Pd and therefore could provide the information on whether it was dependent on support nature. The actual % selectivities to ethylene were estimated by the ratio of the product ethylene concentration to the sum concentration of all other products formed:

$$S(\%) = \frac{(C_2H_4)}{(C_2H_4) + (C_2H_6) + 2(C_4H_6) + 2(C_4H_8) + 2(C_4H_{10})}$$

Table 2 presents actual and normalized conversions of acetylene and actual selectivities to ethylene obtained on supported Pd catalysts which were prepared and used in accordance with this invention.

| Table 2. Supported monometallic Pd catalysts | | | | | | |
|---|---|---|---|---|---|---|
| Example number | Catalyst composition | Catalyst charge (g) | T (°C) | Conversion % %/mg | | Selectivity (%) |
| Comp. 1 | 0.3%Pd/Al$_2$O$_3$ | 0.40 | 100 | 68.0 | 57 | 95.0 |
|  |  |  | 140 | 99.0 | 82 | 94.0 |
| Comp. 2 | 0.5% Pd/Al$_2$O$_3$ | 0.25 | 100 | 62.6 | 50 | 96.2 |
|  |  |  | 140 | 95.2 | 76 | 93.2 |
| 1 | 0.1%Pd/TiO$_2$ | 0.40 | 100 | 35.5 | 89 | 98.2 |
|  |  |  | 140 | 86.7 | 217 | 98.2 |
| 2 | 0.2%Pd/TiO$_2$ | 0.40 | 100 | 71.0 | 89 | 98.5 |
|  |  |  | 140 | 97.9 | 122 | 96.8 |
| 3 | 0.1%Pd/TiO$_2$ | 0.40 | 100 | 45.7 | 114 | 95.7 |
|  |  |  | 140 | 93.0 | 233 | 94.2 |
| 4 | 0.2%Pd/TiO$_2$ | 0.40 | 100 | 77.5 | 97 | 95.9 |
|  |  |  | 140 | 98.6 | 123 | 93.1 |
| 5 | 0.3%Pd/TiO$_2$ | 0.30 | 100 | 43.1 | 48 | 95.9 |
|  |  |  | 140 | 76.7 | 85 | 93.2 |
| 6 | 0.5%Pd/TiO$_2$ | 0.30 | 100 | 59.6 | 40 | 95.5 |
|  |  |  | 140 | 94.2 | 63 | 93.6 |
| 7 | 0.2%Pd/TiO$_2$ | 0.40 | 100 | 84.5 | 106 | 95.3 |
|  |  |  | 140 | 99.3 | 124 | 92.4 |
| 8 | 0.2%Pd/TiO$_2$ | 0.40 | 100 | 62.4 | 78 | 95.3 |
|  |  |  | 140 | 94.2 | 118 | 92.9 |
| 9 | 0.5%Pd/TiO$_2$ | 0.20 | 100 | 68.8 | 69 | 93.5 |
|  |  |  | 140 | 96.7 | 97 | 91.9 |

Examples 1-9 show that good catalysts for acetylene hydrogenation to ethylene were made by depositing palladium on titania. The selectivity to ethylene on these catalysts ranged from 92 to 98%. It showed no correlation with the support texture that comes from the comparison of Examples 4, 7 and 8. 0.2%Pd/TiO$_2$ catalysts prepared in these examples by the same method and supported on titanias with different surface areas and pore 15 volumes gave close selectivities to ethylene. Variation of Pd loading on the same titania support carried out in Examples 3-6 also did not change catalyst selectivity. The factor that affected the selectivity to ethylene was the palladium salt used in catalyst preparation. Catalysts prepared from palladium chloride in Examples 1 and 2 displayed by several percent higher selectivity than the catalysts of the same compositions prepared from palladium nitrate in Examples 3 and 4. The highest selectivity of 98.2-98.5% was obtained on 0.1%Pd/TiO$_2$ and 0.2%Pd/TiO$_2$ catalysts prepared from palladium chloride in Examples 1 and 2. These catalysts were by 2-4% more selective than conventional alumina-supported catalysts tested in Comparative Examples 1 and 2. This can be seen also in Figure 1 where selectivities to ethylene are plotted vs. acetylene conversion. It is seen that TiO$_2$-supported catalysts were indeed more selective than Al$_2$O$_3$-supported catalysts, and that the selectivity to ethylene on both types of catalysts did not depend on Pd loading. We believe that the increase by 2-4% is a significant improvement for the selectivity that values 93% and higher. Since the catalysts listed in Table 2 had different Pd loadings and were tested using different amounts charged to the reactor, the comparison of their activities was made on the basis of acetylene conversion normalized to the weight of palladium in the catalyst charge. This so-called specific activity did not depend on support surface area or gore volume. Such conclusion comes from the comparison of 0.2%Pd/TiO$_2$ catalysts prepared in Examples 4, 7 and 8 by impregnation of different titanias with palladium nitrate. The Pd source also had a little effect on catalyst activity. The relative difference in specific activity of catalysts prepared from palladium nitrate (Examples 3 and 4) and from palladium chloride (Examples 1 and 2) was marginal not exceeding 25%. Palladium loading was found to considerably affect the specific activity of TiO2-supported catalysts. With increasing palladium loading from 0.1 to 0.5% in Examples 3-6, normalized acetylene conversion decreased by 3 times probably due to the decreasing dispersion of palladiuin on the titania surface. The most active was 0.1%Pd/TiO2 prepared in Example 3. The normalized acetylene conversion on this catalyst was by 2-3 times higher than that on conventional 0.3%Pd/Al$_2$O$_3$ catalyst tested in Comparative Example 1. This makes possible to run the process at higher space velocity using catalyst with lower content of precious palladium.

Table 3 reports the results of testing promoted Pd catalysts prepared and used in accordance with the invention. It is seen that bimetallic catalysts were also effective in the liquid-phase acetylene hydrogenation to ethylene.

| Table 3. Supported bimetallic Pd catalysts | | | | | | |
|---|---|---|---|---|---|---|
| Example number | Catalyst composition | Catalyst charge (g) | T (°C) | Conversion % %/mg | | Selectivity (%) |
| 10 | 0.1%Pd-0.1%K/TiO$_2$ | 0.40 | 120 | 69.6 | 174 | 98.3 |
| | | | 140 | 89.4 | 224 | 97.9 |
| 11 | 0.1 %Pd-0.1 %Au/Ti02 | 0.40 | 100 | 40.2 | 101 | 98.0 |
| | | | 140 | 85.2 | 213 | 97.5 |
| 12 | 0.2%Pd-0.2%Au/TiO$_2$ | 0.40 | 100 | 65.6 | 82 | 97.7 |
| | | | 140 | 97.1 | 121 | 96.9 |
| 13 | 0.1%Pd-0.1%K/TiO$_2$ | 0.40 | 100 | 41.8 | 105 | 97.4 |
| | | | 140 | 90.8 | 227 | 96.1 |
| 14 | 0.1%Pd-0.1%Au/Ti02 | 0.40 | 100 | 42.6 | 107 | 97.7 |
| | | | 140 | 84.5 | 211 | 96.9 |
| 15 | 0.2%Pd-0.2%K/TiO$_2$ | 0.40 | 100 | 56.4 | 71 | 96.2 |
| | | | 140 | 93.0 | 116 | 94.4 |
| 16 | 0.2%Pd-0.2%Au/TiO$_2$ | 0.40 | 100 | 58.0 | 73 | 96.4 |
| | | | 140 | 94.5 | 118 | 94.9 |
| 17 | 0.2%Pd-0.2%Ru/TiO$_2$ | 0.40 | 100 | 61.1 | 76 | 91.3 |
| | | | 140 | 98.6 | 123 | 89.4 |
| 18 | 0.2%Pd-0.2%La/TiO$_2$ | 0.40 | 100 | 64.3 | 80 | 92.1 |
| | | | 140 | 95.0 | 119 | 86.6 |
| 19 | 0.2%Pd-0.2%V/TiO$_2$ | 0.40 | 100 | 92.2 | 115 | 92.5 |
| | | | 140 | 99.5 | 124 | 90.5 |
| 20 | 0.2%Pd-0.2%V/TiO$_2$ | 0.40 | 100 | 60.6 | 76 | 93.1 |
| | | | 140 | 99.3 | 124 | 92.4 |

(continued)

| Table 3. Supported bimetallic Pd catalysts | | | | | | |
|---|---|---|---|---|---|---|
| Example number | Catalyst composition | Catalyst charge (g) | T (°C) | Conversion | | Selectivity (%) |
| | | | | % | %/mg | |
| 21 | 0.2%Pd-0.2%Au/TiO$_2$ | 0.41 | 100 | 84.5 | 106 | 95.0 |
| | | | 140 | 97.7 | 122 | 92.4 |
| 22 | 0.2%Pd-0.3%Au/TiO$_2$ | 0.40 | 100 | 83.1 | 116 | 94.3 |
| | | | 140 | 99.0 | 124 | 91.4 |
| 23 | 0.2%Pd-0.3%Ce/TiO$_2$ | 0.40 | 100 | 66.0 | 83 | 94.6 |
| | | | 140 | 97.9 | 122 | 92.1 |
| 24 | 0.2%Pd-0.2%Ni/TiO$_2$ | 0.40 | 100 | 79.8 | 100 | 96.0 |
| | | | 140 | 98.3 | 123 | 94.4 |

At high conversion of acetylene, selectivity to ethylene ranged from 87 to 98%, with the highest obtained on 0.1%Pd-0.1%K/TiO$_2$ and 0.1%Pd-0.1 %Au/TiO2 compositions. Herein catalysts prepared from palladium chloride in Examples 10 and 11 gave slightly higher selectivity than the catalysts prepared from palladium nitrate in Examples 13 and 14. 0.2%Pd-0.2%Au/TiO$_2$ catalyst prepared from palladium chloride in Example 12 was also somewhat more selective than the catalyst of the same composition prepared from palladium nitrate in Example 16, From the comparison of these examples it comes also that the specific activity of bimetallic catalysts did not depend on palladium source. It was mainly determined by the palladium loading. As one can see in Table 3, normalized conversion of acetylene on bimetallic catalysts containing 0.2% Pd was always lower than that on 0.1 %Pd-based bimetallic catalysts.

As for the effect of adding second metal on the behavior of monometallic Pd/TiO$_2$ catalysts, there were different cases. Potassium added in Examples 13 and 15 to the 0.1 %Pd/TiO$_2$ and 0.2%Pd/TiO$_2$ catalysts prepared from palladium nitrate in Examples 3 and 4 slightly decreased specific activity and somewhat increased the selectivity to ethylene. Gold added to the same catalysts in Examples 14 and 16 had stronger effect on selectivity having increased it by 2-3%. Nickel added to the 0.2%Pd/TiO$_2$ catalyst in Example 24 also improved the selectivity by 2% keeping specific activity unchanged. Vanadium and cerium added to the same catalyst in Examples 20 and 23 practically did not change its behavior. On the other hand, ruthenium and lanthanum added in Examples 17 and 18 to the 0.2%Pd/TiO$_2$ catalyst prepared in Example 8 decreased the selectivity by 2-6%. In general, adding second metal had minor effect on behavior of Pd/TiO$_2$ catalysts indicating that palladium was the element responsible of the activity and selectivity of catalysts disclosed in this invention. Using titania as a support improved catalytic behavior of palladium as compared to using alumina. It is known from the open literature (S.J. Tauster and S.C. Fung, Journal of Catalysis, 1978, 55, 29; S.J.Tauster et al., Science, 1981, 211, 1121) that Pd interacts with Ti species stronger than with Al species. Based on that, the promotional effect of titania can be attributed to the case of the so-called "strong metal-support interaction (SMSI)".

This invention with higher specific activity and better selectivity of Pd catalysts supported on titania allows the hydrogenation process to operate at higher space velocity or/and lower temperature to attain a given yield of desired product.

Bimetallic catalysts of Pd and K, B, Au and Ag, respectively, were prepared by impregnation of base catalyst with the salt of second metal, followed by drying and reduction under the standard conditions. First these catalysts were tested with the feed that contained 0.5vol%CO.

The catalysts were tested in the hydrogenation of acetylene dissolved/absorbed in dimethylformamide (DMF). An acetylene solution was first prepared by slowly bubbling pure acetylene at atmospheric pressure into a vessel containing DMF. The DMF saturated with acetylene was then drawn to vertically mounted down-flow ¼ % stainless steel tubular reactor containing catalyst. Co-currently, a gas consisting of 5% CH$_4$, 0.5% CO). 90% H$_2$ and 4.5% He by volume was flowed under pressure and mixed with the liquid stream in the reactor to contact the catalyst. The amount of catalyst loaded in the reactor was 0.3 gram. The catalyst was placed in the reactor center section in a fixed bed configuration. All catalysts were tested at reactor pressure of 200 psig and temperature of 110°C. The liquid reactant flow rate was set at 0.053 ml/min and the gaseous reactant stream was flowed at a rate of 100 ml/min, upon exiting reactor, the reaction effluent cooled and entered the knock-out pot where a gas-liquid separation was made. The overhead gas was analyzed by a gas chromatograph (GC) using CH$_4$ as an internal standard. It usually consisted of C$_2$ and C$_4$ hydrocarbons and CO which did not react. The liquid comprising DMF, C$_2$, C$_4$, and trace C$_6$ hydrocarbons was analyzed by another GC to obtain quantitative composition analysis. The results are listed in Table 4 in comparison with the data on activity and selectivity of the 0.2%Pd/TiO$_2$ catalyst.

Table 4. Activity and selectivity of bimetallic TiO$_2$-supported catalysts at 0.5% CO in the feed (catalyst amount 0.3 g, temperature 110°C, pressure 1379 kPa gauge (200 psig) gas flow rate 100 ml/min, liquid flow rate 0.053 ml/min)

| Metals loading (weight %) | Acetylene conversion (%) | Product selectivity (%) | | |
|---|---|---|---|---|
| | | C$_2$H$_4$ | C$_2$H$_6$ | C$_4$'s |
| 0.2 Pd | 98.8 | 94.5 | 2.2 | 3.4 |
| 0.2 Pd - 0.2 Au | 98.1 | 94.6 | 2.4 | 2.9 |
| 0.2 Pd - 0.2 K | 91.8 | 94.6 | 2.9 | 2.5 |
| 0.2 Pd - 0.4 B | 91.0 | 93.5 | 2.9 | 2.5 |
| 0.2 Pd - 0.3 Ag | 58.6 | 96.7 | 1.7 | 1.6 |

The data above demonstrates the efficacy of a palladium-based catalyst with and without metal modifiers in a process for partial hydrogenation of acetylene to ethylene in the presence of carbon monoxide. In one embodiment of the catalyst, metal modifiers are essentially not present in the catalyst. One example of the catalyst is 0.2%Pd/TiO$_2$. Carbon monoxide may be present in the range up to 5vol%.

Bimetallic catalysts were also tested with the feed that did not contain CO. in these experiments carbon monoxide in the gas stream was replaced with helium to keep gas total flow rate unchanged. The results are presented in Table 5.

Table 5. Activity and selectivity of bimetallic TiO$_2$-supported catalysts at no CO in the feed (catalyst amount 0.3 g, temperature 110°C, pressure 1379 kPa gauge (200 psig), gas flow rate 100 ml/min, liquid flow rate 0.053 ml/min)

| Metals loading (weight %) | Acetylene conversion (%) | Product selectivity (%) | | |
|---|---|---|---|---|
| | | C$_2$H$_4$ | C$_2$H$_6$ | C$_4$'s |
| 0.2 Pd | 98.1 | 43.5 | 48.1 | 8.4 |
| 0.2 Pd - 0.2 Ga | 99.9 | 29.1 | 59.4 | 11.5 |
| 0.2 Pd - 0.2 Au | 97.2 | 41.2 | 48.7 | 10.1 |
| 0.2 Pd - 0.2 K | 99.5 | 56.8 | 31.2 | 12.0 |
| 0.2 Pd - 0.4 B | 98.3 | 59.7 | 29.5 | 10.8 |
| 0.2 Pd - 0.3 Ag | 99.1 | 64.6 | 27.2 | 8.3 |

The data above demonstrates the efficacy of a palladium-based catalyst having metal modifiers, such as potassium, boron and silver, in a process for partial hydrogenation of acetylene to ethylene in the absence of carbon monoxide. In the absence of CO, selectivity to ethylene was, however; essentially lower than in the presence of CO (see Table 4). The highest selectivity to ethylene in the absence of CO was displayed on the Ag-modified Pd catalyst. In some embodiments of the catalyst, the metal modifier is silver and the catalyst may contain 0.3 to 0.9 weight % of silver. One example of the catalyst is 0.2%Pd-0.9%Ag/TiO$_2$. In the context of the present invention, the phrase "in the absence of carbon monoxide" shall mean carbon monoxide nay be present in the range up to 0. 1 vol%.

In an embodiment, a process for the selective hydrogenation acetylene in a hydrocarbon-containing feed, said process can comprise: contacting the hydrocarbon-containing feed in the liquid phase with a catalyst comprising palladium supported on titania.

In the various processes, (i) the catalyst further comprises at least one metal from Group IA, IB, IIIA, IIIB, VB, or VIIB; and/or (ii) the at least one metal is present at greater than 0 to about 0.5% by weight; and/or (iii) the at least one metal is from lithium, potassium, cesium, copper, silver, gold, a lanthanide, vanadium, niobium, tantalum, cobalt, boron, nickel, ruthenium, rhodium, or platinum; and/or (iv) the catalyst consists essentially of palladium supported on titania; and/or (v) the palladium is present at 0.05 to 1% by weight; and/or (vi) the palladium is present at 0.1 to 0.5% by weight; and/or (vii) the catalyst comprises at least one metal from potassium, gold, silver, boron, lanthanum, cerium, vanadium, nickel, or ruthenium; and/or (viii) the feed further comprises carbon monoxide in the range up to 5 vol%; and/or (ix) the hydrocarbon-containing feed comprises acetylene and non-hydrocarbon solvent/absorbent; and/or (x) the solvent/absorbent is dimethylformamide, and/or (xii) the titania has total pore volume of at least 0.25 cm$^3$/g, an average pore diameter of greater than 150 angstroms, BET surface area in the range of 30 to 100 m$^2$/g.

In various embodiments, (i) the feed contains essentially no carbon monoxide and the catalyst consists essentially of palladium and at least one metal from Group IA, IB, IIIA, IIIB, VB, or VIIB, supported on titania; and/or (ii) the at least one metal is present at greater than 0 to about 0.5% by weight; and/or (iii) the palladium is present at 0.05 to 1% by weight; and/or (iv) the palladium is present at 0.1 to 0.5% by weight; and/or (v) the hydrocarbon-containing feed comprises an alkyne and non-hydrocarbon solvent/absorbent; and/or (vi) the solvent/absorbent is and/or (viii) the titania has total pore volume of at least 0.25 cm$^3$/g, an average pore diameter of greater than 150 angstroms, BET surface area in the range of 30 to 100 m$^2$/g.

## Claims

1. A process for the selective hydrogenation of acetylene to ethylene, said process comprising: contacting a hydrocarbon-containing feed in the liquid phase with a catalyst comprising palladium supported on titania, wherein the feed comprises the acetylene and dimethylformamide, and
liquid phase hydrogenating the acetylene.

2. The process of Claim 1, wherein the catalyst further comprises at least one metal from Group IA, IB, IIIA, IIIB, VB, or VIIB.

3. The process of Claim 2, wherein the at least one metal is present at greater than 0 to about 0.5% by weight.

4. The process of any of Claims 2 - 3, wherein the at least one metal is from lithium, potassium, cesium, copper, silver, gold, a lanthanide, vanadium, niobium, tantalum, cobalt, boron, nickel, ruthenium, rhodium, or platinum.

5. The process of any of Claims 2 - 3, wherein the catalyst consists essentially of palladium supported on titania.

6. The process of Claim 5, wherein palladium is present at 0.05 to 1% by weight.

7. The process of Claim 6, wherein palladium is present at 0.1 to 0.5% by weight.

8. The process of any of Claims 2 - 3, wherein the catalyst comprises at least one metal from potassium, gold, silver, boron, lanthanum, cerium, vanadium, nickel, or ruthenium.

9. The process of any of Claims 1 - 8, wherein the feed further comprises carbon monoxide in the range up to 5 vol%.

10. The process of Claim 1, wherein the feed contains essentially no carbon monoxide and the catalyst consists essentially of palladium and at least one metal from Group IA, IB, IIIA, IIIB, VB, or VIIB, supported on titania.

11. The process of Claim 10, wherein palladium is present at 0.05 to 1% by weight.

12. The process of Claim 11, wherein palladium is present at 0.1 to 0.5% by weight.

13. The process of any of Claims 1 - 12, wherein the titania has total pore volume of at least 0.25 cm$^3$/g, an average pore diameter of greater than 150 angstroms, BET surface area in the range of 30 to 100 m$^2$/g.

## Patentansprüche

1. Ein Verfahren zur selektiven Hydrierung von Acetylen zu Ethylen, wobei das Verfahren folgendes umfasst: In-Kontaktbringen einer Kohlenwasserstoff-enthaltenden Einspeisung in der flüssigen Phase mit einem Katalysator umfassend Palladium auf Titandioxid-Träger, worin die Einspeisung das Acetylen und Dimethylformamid umfasst; und
Flüssigphasenhydrierung des Acetylens.

2. Das Verfahren gemäß Anspruch 1, worin der Katalysator weiter mindestens ein Metall aus Gruppe IA, IB, IIIA, IIIB, VB, oder VIIB umfasst.

**3.** Das Verfahren gemäß Anspruch 2, worin das mindestens eine Metall zu mehr als 0 bis ungefähr 0,5 Gewichtsprozent vorhanden ist.

**4.** Das Verfahren gemäß irgendeinem der Ansprüche 2-3, worin das mindestens eine Metall aus Lithium, Kalium, Cäsium, Kupfer, Silber, Gold, einem Lanthanoid, Vanadium, Niobium, Tantal, Cobalt, Bor, Nickel, Ruthenium, Rhodium, oder Platin ist.

**5.** Das Verfahren gemäß irgendeinem der Ansprüche 2-3, worin der Katalysator im Wesentlichen aus Palladium auf Titandioxid-Träger besteht.

**6.** Das Verfahren gemäß Anspruch 5, worin Palladium zu 0,05 bis 1 Gewichtsprozent vorhanden ist.

**7.** Das Verfahren gemäß Anspruch 6, worin Palladium zu 0,1 bis 0,5 Gewichtsprozent vorhanden ist.

**8.** Das Verfahren gemäß irgendeinem der Ansprüche 2-3, worin der Katalysator mindestens ein Metall von Kalium, Gold, Silber, Bor, Lanthan, Cer, Vanadium, Nickel, oder Ruthenium umfasst.

**9.** Das Verfahren gemäß irgendeinem der Ansprüche 1-8, worin die Einspeisung weiter Kohlenstoffmonoxid in dem Bereich bis zu 5 Volumenprozent umfasst.

**10.** Das Verfahren gemäß Anspruch 1, worin die Einspeisung im Wesentlichen kein Kohlenstoffmonoxid enthält und der Katalysator im Wesentlichen aus Palladium und mindestens einem Metall aus Gruppe IA, IB, IIIA, IIIB, VB, oder VIIB, auf Titandioxid-Träger, besteht.

**11.** Das Verfahren gemäß Anspruch 10, worin Palladium zu 0,05 bis 1 Gewichtsprozent vorhanden ist.

**12.** Das Verfahren gemäß Anspruch 11, worin Palladium zu 0,1 bis 0,5 Gewichtsprozent vorhanden ist.

**13.** Das Verfahren gemäß irgendeinem der Ansprüche 1-12, worin das Titandioxid ein Gesamtporenvolumen von mindestens 0,25 cm$^3$/g, einen durchschnittlichen Porendurchmesser von größer als 150 Ångström, eine BET Oberflächenfläche im Bereich von 30 bis 100 m$^2$/g hat.

## Revendications

**1.** Procédé d'hydrogénation sélective d'acétylène en éthylène, ledit procédé comprenant : la mise en contact d'une alimentation contenant un hydrocarbure dans la phase liquide avec un catalyseur comprenant du palladium supporté sur du dioxyde de titane, dans lequel l'alimentation comprend de l'acétylène et du diméthylformamide, et l'hydrogénation en phase liquide de l'acétylène.

**2.** Procédé selon la revendication 1, dans lequel le catalyseur comprend en outre au moins un métal du groupe IA, IB, IIIA, IIIB, VB ou VIIB.

**3.** Procédé selon la revendication 2, dans lequel l'au moins un métal est présent à plus de 0 jusqu'à environ 0,5 % en poids.

**4.** Procédé selon l'une quelconque des revendications 2 à 3, dans lequel l'au moins un métal est choisi parmi le lithium, le potassium, le césium, le cuivre, l'argent, l'or, un lanthanide, le vanadium, le niobium, le tantale, le cobalt, le bore, le nickel, le ruthénium, le rhodium ou le platine.

**5.** Procédé selon l'une quelconque des revendications 2 à 3, dans lequel le catalyseur se compose essentiellement de palladium supporté sur du dioxyde de titane.

**6.** Procédé selon la revendication 5, dans lequel le palladium est présent à hauteur de 0,05 à 1 % en poids.

**7.** Procédé selon la revendication 6, dans lequel le palladium est présent à hauteur de 0,1 à 0,5 % en poids.

**8.** Procédé selon l'une quelconque des revendications 2 à 3, dans lequel le catalyseur comprend au moins un métal choisi parmi le potassium, l'or, l'argent, le bore, le lanthane, le cérium, le vanadium, le nickel ou le ruthénium.

9. Procédé selon l'une quelconque des revendications 1 à 8, dans lequel l'alimentation comprend en outre du monoxyde de carbone dans la plage allant jusqu'à 5 % en volume.

10. Procédé selon la revendication 1, dans lequel l'alimentation ne contient sensiblement pas de monoxyde de carbone et le catalyseur comprend essentiellement du palladium et au moins un métal du groupe IA, IB, IIIA, IIIB, VB ou VIIB, supporté sur du dioxyde de titane.

11. Procédé selon la revendication 10, dans lequel le palladium est présent à hauteur de 0,05 à 1 % en poids.

12. Procédé selon la revendication 11, dans lequel le palladium est présent à hauteur de 0,1 à 0,5 % en poids.

13. Procédé selon l'une quelconque des revendications 1 à 12, dans lequel le dioxyde de titane a un volume de pore total d'au moins 0,25 cm$^3$/g, un diamètre de pore moyen supérieur à 150 angströms, une surface BET comprise dans la plage allant de 30 à 100 m$^2$/g.

Figure 1. Selectivity comparison for Pd catalysts supported on alumina and titania
(experiments were performed at temperatures 100-140°C, pressure 200 psig,
liquid stream flow rate 0.053 ml/min and gas flow rate 100 ml/min) 1379 kPa gauge (200 psig)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4128595 A
- US 4571442 A
- US 5059732 A
- US 6867338 B
- RU 2169755
- US 6358399 B
- US 7045670 B
- US 7220701 B
- US 7301062 B
- US 6388150 B
- US 4691070 A
- WO 0064846 A

**Non-patent literature cited in the description**

- **R.K. EDVINSOON et al.** *Ind. Eng. Chem. Res.,* 1995, vol. 34, 94-100
- **H. MARWAN ; J.M. WINTERBOTTOM.** *Catalysis Today,* 2004, vol. 97, 325-330
- **S.J. TAUSTER ; S.C. FUNG.** *Journal of Catalysis,* 1978, vol. 55, 29
- **S.J.TAUSTER et al.** *Science,* 1981, vol. 211, 1121